# EUROPÄISCHE PATENTSCHRIFT

(11) **EP 2 788 289 B1**
(45) Veröffentlichungstag und Bekanntmachung des Hinweises auf die Patenterteilung: **31.07.2019**
(21) Anmeldenummer: 12784210.2
(22) Anmeldetag: 10.11.2012
(51) Int. Cl.: C01D 7/12, C01D 7/26, C01D 7/35, A61K 47/08

(54) **PORENARMES, WASSERFREIES NATRIUMCARBONAT**
ANHYDROUS SODIUM CARBONATE WITH FEW PORES
CARBONATE DE SODIUM ANHYDRE FAIBLEMENT POREUX

(30) Priorität: 09.12.2011 EP 11009721
(43) Veröffentlichungstag der Anmeldung: 15.10.2014
(73) Patentinhaber: Merck Patent GmbH, 64293 Darmstadt (DE)
(72) Erfinder: KLATYK, Jens, 64347 Griesheim (DE); PETH, Hans-Kurt, 64665 Alsbach-Haehnlein (DE); WEDEL, Thorsten, 64589 Stockstadt/Rhein (DE); MODDELMOG, Guenter, 64354 Reinheim (DE)
(86) Internationale Anmeldenummer: PCT/EP2012/004679
(87) Internationale Veröffentlichungsnummer: WO 2013/083226

(56) Entgegenhaltungen:
- CN-A- 101 712 480
- DE-A1- 2 926 380
- DE-T1- 670 160
- JP-B- 46 026 101
- US-B2- 6 710 050

## Beschreibung

Die vorliegende Erfindung betrifft ein hochreines, porenarmes, wasserfreies Natriumcarbonat zur Verwendung in pharmazeutischen Formulierungen und in der Lebensmittelindustrie. Weiterhin wird ein neues Verfahren zur Herstellung dieses Natriumcarbonats zur Verfügung gestellt.

### Stand der Technik

Wasserfreies Natriumcarbonat ("kalciniertes Soda") wird für eine Vielzahl technischer, pharmazeutischer und lebensmitteltechnologischer Prozesse und in den verschiedensten Formulierungen eingesetzt. In diesen Rezepturen wirkt es u.a. alkalisierend - aber häufig wird es auch wegen seiner Eigenschaft mit verdünnten Säuren eine Kohlendioxidfreisetzung zu entwickeln in der Rezepturentwicklung von "brausenden" Präparaten berücksichtigt.

Für die Herstellung von wasserhaltigem Natriumcarbonat sind verschiedene Verfahren bekannt. Dabei fällt das Natriumcarbonat als Deka-, Hepta- oder Monohydrat an. Wasserfreies Natriumcarbonat wird üblicherweise durch Kalzinieren oder Erhitzen des Monohydrats gewonnen.

Das älteste Verfahren zur Herstellung von wasserfreiem Natriumcarbonat wurde 1790 von Leblanc entwickelt. Darin wird aus Natriumsulfat durch Erhitzen mit Kalk und Kohle das Na₂CO₃, CaS und Kohlendioxid erhalten. Das Natriumcarbonat wird durch Auslaugen aus dem Reaktionsprodukt abgetrennt. Nachteile dieses Verfahrens sind die als Nebenprodukte anfallenden CaS und HCl sowie der hohe Energieverbrauch. Dieses Verfahren wurde durch das 1861 entwickelte Solvay-Verfahren abgelöst. Hierbei wird in eine nahezu gesättigte Kochsalzlösung zuerst Ammoniak und anschließend Kohlendioxid eingeleitet. Dadurch wird das schwerlösliche Natriumhydrogencarbonat gebildet. Durch Erhitzen erhält man aus dem abgetrennten Natriumcarbonat-Monohydrat das gewünschte wasserfreie Natriumcarbonat.

Durch Toyo Soda wird in DE 29 26 380 A1 ein Verfahren beschrieben wonach wasserfreies Natriumcarbonat aus einer konzentrierten Lösung eines Gemisches aus Natriumchlorid und Natriumcarbonat hergestellt wird. Das gewünschte Natriumcarbonat wird aus der konzentrierten Lösung auskristallisiert.

Durch Tianjin Chemical Reagent Res. wiederum wird in CN101712480A ein ähnliches Verfahren beschrieben, worin störende Metallionen wie Mg²⁺, Ca²⁺ und Fe²⁺ durch Zugabe von Wasserstoffperoxid aus Natriumcarbonatlösungen entfernt werden. Natriumcarbonatkristalle werden durch Filtrieren entfernt. Das erhaltene kristalline Produkt wird anschließend getrocknet. Auch wenn durch die letztere Methode die Reinheit des Industrieprodukts verbessert werden soll, ist das Produkt nicht für die Herstellung von pharmazeutischen Produkten geeignet.

Wegen des geringen Energiebedarfs wird vermehrt die Gewinnung von Natriumcarbonat aus natürlichen Quellen bevorzugt. Natürliche Vorkommen sind z. B. aus Trona (USA), den großen Salzseen in Ägypten (Wadi Natrum) und Nord- und Südamerika bekannt. Das gewünschte wasserfreie Natriumcarbonat wird daraus durch Löse-, Reinigungs- und Eindampf-, bzw. Kalzinierprozesse gewonnen.

So ist aus dem japanischen Patent JP 46026101B4 ein Verfahren bekannt, worin eine Natriumcarbonatlösung hergestellt wird, die mit NaOH versetzt wird. In die so erhaltene Lösung wird CO₂-Gas eingeleitet. Durch eine Vakuumverdampfung wird eine gesättigte Lösung erhalten, aus der wiederum das Monohydrat auskristallisiert wird, wenn weiteres NaOH zugegeben wird. Nachteil dieses Verfahrens ist, dass einerseits CO₂-Gas eingeleitet werden muss, um eine Auskristallisation des Monohydrats zu erreichen. Andererseits muss nach der Abtrennung eine getrennte Kalzinierung erfolgen um das wasserfreie Natriumcarbonat zu erhalten.

Wasserfreies Natriumcarbonat ist hygroskopisch und kann selbst bis zu 10 Gew.% Wasser aus der Umgebung binden ohne feucht auszusehen. Dabei kommt es zur erneuten Bildung des Monohydrats. Dieses ausgeprägte Wasserbindevermögen des wasserfreien Natriumcarbonats kann zu Stabilitätsproblemen in pharmazeutischen Rezepturen führen - insbesondere wenn feuchtigkeitsempfindliche Substanzen enthalten sind. Daneben kann es durch das aufgenommene Wasser zu Verfärbungsreaktionen kommen.

Ein erhebliches Problem ergibt sich jedoch durch eine unerwünschte Kohlendioxidentwicklung in Gegenwart von sauer reagierenden Rezepturkomponenten. Insbesondere in sogenannten Brauseformulierungen kann diese vorzeitige Kohlendioxidentwicklung zu einem Druckaufbau in Fertigpackmitteln und zu deren Zerstörung führen ("Bombagen").

### Aufgabenstellung

Es ist daher Aufgabe der vorliegenden Erfindung, ein kostengünstiges, in einfacher Weise energiesparend durchführbares Verfahren zur Herstellung von hochreinem, wasserfreiem Natriumcarbonat zur Verfügung zu stellen, wodurch ein Produkt zur Verfügung gestellt werden kann, das die aufgezählten Nachteile nicht aufweist, das lagerstabil ist und aufgrund seines morphologischen Aufbaus in Gegenwart von Säuren weniger zur Freisetzung von CO₂ neigt und das eine im Vergleich zu handelsüblichen wasserfreien Natriumcarbonaten geringere Hygroskopizität besitzt.

### Kurze Beschreibung der Erfindung

Es wurde gefunden, dass durch ein synthetisches Herstellverfahren ein kristallines Natriumcarbonat erhalten wird, welches sich durch einen besonders niedrigen Wassergehalt auszeichnet und daher als wasserfrei bezeichnet werden kann und welches eine besonders geringe Oberfläche bei einem sehr geringen Porenvolumen besitzt. In REM-Aufnahmen zeigt sich, dass das nach dem neu entwickelten Verfahren hergestellte wasserfreie Natriumcarbonat eine besonders kompakte Oberflächenstruktur aufweist im Vergleich zu herkömmlichen, im Handel erhältlichen wasserfreiem Natriumcarbonat. Diese neuen Eigenschaften führen zu einer verbesserten Kompatibilität mit anderen, insbesondere mit Hydrolyse-empfindlichen, Hilfs- und Wirkstoffen bei gleichzeitig deutlich reduzierter Hygroskopizität.

Insbesondere zeichnet sich das wasserfreie Natriumcarbonat dadurch aus, dass es
a) aus Teilchen mit einer glatten, porenarmen Oberflächenstruktur besteht,
b) eine BET-Oberfläche von kleiner 1m²/g, vorzugsweise von kleiner 0.5 m²/g, insbesondere bevorzugt von kleiner 0,2 m²/g besitzt
   und
c) nach seiner Herstellung einen Trocknungsverlust von nicht mehr als 0,2 Gew.-%, vorzugsweise von weniger als 0,15 Gew.-% besitzt. Weiterhin weist es ein Wasserdampfaufnahmevermögen (DVS) von <5 Gew.-% bei 60% relative Feuchte; < 10 Gew.-% bei 70% relative Feuchte; <15 Gew.-% bei 80% relative Feuchte und < 40 Gew.-% bei 90% relative Feuchte auf. Durch acidimetrische Bestimmung wurde für das erfindungsgemäße wasserfreie Natriumcarbonat ein Gehalt an Natriumcarbonat von 99,5 bis 100% ermittelt. Somit kann dem Anwender ein hochreines, wasserfreies Natriumcarbonat zur Verfügung gestellt werden, welches im Vergleich zu herkömmlichen Produkten wesentlich verbesserte Eigenschaften aufweist, insbesondere wenn es für die Herstellung von pharmazeutischen Formulierungen verwendet wird.

Zur Durchführung des erfindungsgemäßen Verfahrens zur Herstellung dieses neuen wasserfreien Natriumcarbonats mit verbesserten Eigenschaften wird eine wässrige Lösung hergestellt, worin
a) Natriumhydrogencarbonat (NaHCO₃) und Natronlauge (NaOH) in einem molaren Verhältnis von 1 : 1 bis 1 : 1,3 gelöst sind
   und
b) Natriumhydrogencarbonat und Natronlauge in einer solchen Menge gelöst sind, dass die Lösung bei 65 °C eine Dichte im Bereich von 1,2 bis 1,4 g/ml, bevorzugt im Bereich von 1,25 bis 1,34 g/ml, besonders bevorzugt im Bereich von 1,28 bis 1,31 g/ml aufweist,
c) die Lösung nacheinander mit einer katalytischen Menge Wasserstoffperoxid und mit Aktivkohle versetzt wird und nach ausreichender Durchmischung filtriert wird,
   und
d) die erhaltene klare Lösung einer Kristallisationsapparatur zugeführt wird, worin durch Vakuumverdampfung bei erhöhter Temperatur Flüssigkeit entfernt wird und eine Supensionsdichte im Bereich von 1,54 - 1,86 g/ml, bevorzugt 1,58 - 1,82 g/ml, besonders bevorzugt im Bereich von 1,62 - 1,78 g/ml eingestellt wird, bei der die Kristallisation erfolgt,
e) die gebildeten Kristalle durch Filtrieren, Zentrifugieren oder Abschleudern abgetrennt werden
   und
f) bei einer Temperatur im Bereich von 60 -70 °C in einen Trockner gefüllt werden und unter fortwährender Durchmischung bei einer Temperatur von 115 bis 125 °C getrocknet werden bis das erhaltene Produkt einen Trocknungsverlust von nicht mehr als 0,2 Gew.-% aufweist.
Gute Kristallisationsergebnisse werden erzielt, wenn die Temperatur während der Kristallisation im Bereich von 50 bis 95 °C eingestellt wird und die Dichte der Suspension im Bereich von 1,54 - 1,86 g/ml liegt, insbesondere wenn die Temperatur im Bereich von 55 bis 90 °C eingestellt wird und die Dichte der Suspension im Bereich von 1,62 bis 1,78 g/ml liegt.

Vorzugsweise wird die Kristallisation in einem Schlaufenkristaller durchgeführt. In einer besonderen Ausführungsform wird das erhaltene Kristallisat aus dem Kristaller mittels Mischerschnecke auf eine Siebschleuder geführt, mit Wasser gewaschen und über einen Stromtrockner in einen Mischertrockner gefüllt.

In einer bevorzugten Ausführungsform wird das Kristallisat über eine Schneckenförderung in einen Fließbetttrockner geführt und bei einer Temperatur im Bereich von 175 bis 200 °C, vorzugsweise im Bereich von 180 bis 195 °C, insbesondere bevorzugt bei 187°C, mit einer ausreichenden Verweilzeit getrocknet, dass das erhaltene Produkt einen Trocknungsverlust von nicht mehr als 0,2 Gew.-% aufweist.

Je nach gegebenen Voraussetzungen kann die Kristallisation batchweise oder kontinuierlich durchgeführt werden. Vorzugsweise wird die Kristallisation kontinuierlich durchgeführt, womit günstigere Kristallisationsbedingungen ausgenutzt werden können.

Erfindungsgemäß hergestelltes wasserfreies Natriumcarbonat ist besonders geeignet für die Verwendung in galenischen Zubereitungen, insbesondere in ethischen und OTC- Formulierungen. Es kann in Brauseformulierungen, enthaltend Vitamine und/oder Mineralstoffe und/oder Spurenelemente und/oder Hydrolyse-empfindliche Wirkstoffe eingesetzt werden und führt zu verbesserten Eigenschaften dieser Produkte, wie z. B. verbesserte Langzeitstabilität. Besonders geeignet ist das erfindungsgemäße wasserfreie Natriumcarbonat zur Verwendung in Brauseformulierungen, welche sauer wirkende Wirkstoffe und/oder hydrolyseempfindliche Wirkstoffe, wie Vitamin C oder Acetylsalicylsäure, enthalten, weil es wesentlich weniger dazu neigt, in Gegenwart von sauren Additiven oder sauren Wirkstoffen CO₂ abzuspalten. Dabei kann das erfindungsgemäße wasserfreie Natriumcarbonat sowohl im unveränderten als auch im veränderten Zustand zur Anwendung kommen. Unter verändertem Zustand können Änderungen in der Kornverteilung, z. B. durch Siebfraktionierung oder durch Vermahlung, auch Änderungen an der Materialoberfläche, wie z. B. durch das Aufbringen von Überzügen sowie andere Manipulationen gehören. Die verbesserten Eigenschaften haben den Vorteil, dass hergestellte Formulierungen bessere Langzeitstabilitäten aufweisen, und es zu einem wesentlich geringeren Druckaufbau in geschlossenen Verpackungen, wie z. B. in Arzneimittelröhrchen, luftdicht verschlossenen Tütchen oder Blisterverpackungen kommt.

Durch diese Eigenschaften erhält der Formulierer in der pharmazeutischen Industrie, in der Lebensmittelindustrie oder auch in technischen Bereichen ein Material welches die Stabilität seines Endproduktes verbessert. Zusätzlich wird die Entwicklung von Kohlendioxid in Gegenwart von Säuren aus Formulierungen verlangsamt. Dieses hat, wie oben bereits erwähnt, eine verbesserte Lagerstabilität des verpackten Fertigprodukts zur Folge.

Demzufolge besitzt das erfindungsgemäß hergestellte Produkt im Vergleich zu handelsüblicher Ware besonders vorteilhafte Eigenschaften. Es handelt sich dabei um ein synthetisch hergestelltes hochreines, wasserfreies Natriumcarbonat, das die Anforderungen der PhEur (Pharmacopoea Europaea), BP (British Pharmacopoeia), NF (USP/NF Compendiums) erfüllt und einen Gehalt von nicht weniger als 99,5 Gew.-% Natriumcarbonat, sowie einen Trocknungsverlust von nicht mehr als 0,2 Gew.-% aufweist. Dieser Gehalt lässt sich acidimetrisch bestimmen und ist auf getrocknete Substanz bestimmt. Insbesondere handelt es sich um ein Produkt mit sehr niedrigem Wassergehalt, das zwar eine gröbere Kornstruktur aufweist, aber auch eine deutlich höhere Schütt-und Stampfdichte besitzt. Der geringere Fließneigungswinkel des Produkts kann als Indikator für ein verbessertes Fließverhalten der Pulvermasse betrachtet werden. Die Auswertung der REM-Aufnahmen von Produktproben zeigt, dass das erfindungsgemäß hergestellte Natriumcarbonat eine deutliche glattere Kristalloberfläche und damit eine deutlich geringere BET-Oberfläche aufweist als handelsübliche Ware, verbunden mit einem deutlich geringeren Porenvolumen, wie die Werte der Beispiele 1 und 2 in Tabelle 3 zeigen. Letzteres lässt sich durch Quecksilberintrusion bestimmen.

Das Material zeigt eine deutlich geringere Hygroskopizität als bekanntes Natriumcarbonat und hat direkt nach der Herstellung einen extrem geringen Wassergehalt ohne besonderen Trocknungsschritt. Wie Messungen ergeben haben, beträgt der Wassergehalt direkt nach der Herstellung des Produkts nicht mehr als 0,2 Gew.-%, vorzugsweise niedriger als 0,15 Gew.-%.

Das erfindungsgemäße Produkt weist eine glatte Oberfläche auf. Durch REM-Aufnahmen zeigt sich, dass es gleichzeitig eine geringe Porosität besitzt. Beides resultiert in einem verringerten Wasseraufnahmevermögen.

Hieraus ergeben verschiedene vorteilhafte Eigenschaften.
Beispielsweise reagiert das Material in galenischen Formulierungen deutlich langsamer, insbesondere mit hydrolyseempfindlichen Substanzen und weist dadurch bedingt eine verbesserte Lagerstabilität auf.

Durch die im Vergleich mit herkömmlichem Natriumcarbonat verzögerte Wasseraufnahme wird insbesondere in Formulierungen mit einer sauer reagierenden Komponente die Entwicklung gasförmigen Kohlendioxids verzögert und damit eine vorzeitige Aufblähung z.B. von Blisterverpackungen oder das Absprengen von Stopfen aus Röhrenverpackungen vermieden.

In Brausepulvern oder Braustabletten wird durch die verlangsamte Reaktion in Wasser mit der sauren Rezepturkomponente, z.B. mit Zitronensäure, auch eine Verlangsamung der Kohlendioxidbildung erzielt und so ein zu heftiges Überschäumen der trinkfertigen Lösung vermieden.

### Herstellverfahren:

Bisher wird Natriumcarbonat durch eine diskontinuierliche Kristallisation hergestellt. Nach dem erfindungsgemäßen neuen Verfahren kann die Kristallisation ebenfalls batchweise durchgeführt werden, jedoch gestalten sich die Bedingungen wesentlich vorteilhafter, wenn das Verfahren im Gegensatz zu herkömmlichen Verfahren kontinuierlich durchgeführt wird, und insbesondere eine kontinuierliche Kristallisation erfolgt. Auf diese Weise ist es möglich, die Kristallisation unter laufend gleichen Bedingungen ablaufen zu lassen, so dass ein immer gleiches Produkt erhalten wird. Vorzugsweise erfolgt die Herstellung kontinuierlich in einem Schlaufenkristaller. Im Folgenden wird die Herstellung entsprechender Produkte beschrieben und durch die gegebenen Beispiele 1 und 2 näher verdeutlicht. Die gegebenen Beispiele unterscheiden sich insbesondere durch die Art der Endtrocknung/Kalzinierung. An sich handelt es sich um eine Kalzinierung unter milden Bedingungen, da dieser Verfahrensschritt bei recht niedrigen Temperaturen erfolgt. In dieser Phase wird aus dem primär gewonnenen Natriumcarbonat-Hydrat das gewünschte wasserfreie Natriumcarbonat gebildet. In diesem Zusammenhang bedeutet Kalzinierung unter milden Bedingungen, dass Wasserabspaltung erfindungsgemäß bei Temperaturen von etwa 110 bis etwa 200 °C erfolgt, je nach Ausgestaltung des Trockners und der gewünschten Trocknungszeit, während üblicherweise die Kalzinierung unter Wasserabspaltung vorzugsweise bei einigen hundert Grad, d. h. bei 300 bis 400°C bzw. mehr erfolgt.

Zur Durchführung des Verfahrens wird eine wässrige Lösung aus möglichst reinem Natriumcarbonat und Natronlauge hergestellt. Aus dieser Lösung wird in einem ersten Schritt unter geeigneten Bedingungen Natriumcarbonat als Monohydrat auskristallisiert. Zu diesem Zweck wird eine Mutterlauge hergestellt, worin Natriumhydrogencarbonat und Natronlauge in einem molaren Verhältnis von 1 : 1 bis 1 : 1,3 , vorzugsweise in einem Verhältnis von 1 : 1,1 gelöst sind. Das heißt, dass insbesondere durch einen geringen Überschuß an Natronlauge eine verbesserte Auskristallisation erzielt werden kann.
Zur Beschleunigung der Reaktion

NaHCO₃ + NaOH → Na₂CO₃ x H₂O

wird zu dem Reaktionsgemisch eine verhältnismäßig geringe Menge H₂O₂-Lösung (30 %ig) zugefügt, und zwar hat sich herausgestellt, dass eine Zugabe im Verhältnis Natriumhydrogencarbonat/Natronlösung zu H₂O₂-Lösung (30 %ig) im Bereich von 700 : 1 bis 900 : 1, vorzugsweise 800 : 1 bis 760 : 1 insbesondere bevorzugt 780 :1 bezogen auf das Gewicht der Gesamtlösung besonders vorteilhaft ist. Die Lösung wird unter Rühren für einige Minuten, vorzugsweise 10 bis 15 Minuten, erhitzt und für die weitere Verarbeitung wieder auf eine geeignete Temperatur im Bereich von 50 bis 70°C abgekühlt. Diese Lösung lässt man für einige Stunden ruhen bis die Reaktion beendet ist. Nach etwa 6 bis 10 Stunden, vorzugsweise nach 8 Stunden kann die Lösung weiterverarbeitet werden. Im Anschluss an die Ruhezeit wird die so erhaltene Lösung mit Aktivkohle in einem Gewichtsverhältnis von Natriumhydrogencarbonat/Natronlösung zu Aktivkohle in einem Verhältnis von 20000 : 1 bis 10000 : 1, vorzugsweise 16500 : 1 bis 14000 :1, insbesondere bevorzugt 15600 :1, bezogen auf das Gewicht der Gesamtlösung versetzt und intensiv vermischt. Nach ausreichender Durchmischung unter Rühren wird die Lösung mit einer geeigneten Vorrichtung filtriert, vorzugsweise unter Verwendung von Filteranlagen, welche Feinfilter mit einer Porenweite von 1 µm aufweisen.

Durch die in der Lösung gelöste Gewichtsmenge an Natriumhydrogencarbonat und Natronlauge weist die Lösung bei 65 °C eine Dichte im Bereich von 1,2 bis 1,4 g/ml, bevorzugt im Bereich von 1,25 bis 1,34 g/ml, besonders bevorzugt im Bereich von 1,28 bis 1,31 g/ml auf.

Diese filtrierte, klare Lösung wird der Kristallisationsapparatur zugeführt. Vorzugsweise handelt es sich dabei um einen heizbaren Schlaufenkristaller mit Füllstandsregelung, worin die Lösung gerührt werden und mit einer Pumpe umgepumpt werden kann. Insbesondere ist es möglich, den Kristaller mit Vakuum zu beaufschlagen, so dass die Kristallisation kontinuierlich unter Vakuumverdampfung durchgeführt werden kann. Zur Durchführung der Kristallisation stellt sich durch Abdampfen von Wasser in Abhängigkeit von der eingestellten Temperatur eine Suspensionsdichte im Bereich von 1,54 - 1,86 g/ml, bevorzugt 1, 58 - 1,82 g/ml, besonders bevorzugt im Bereich von 1,62 - 1,78 g/ml ein. Wie in Beispiel 1 beschrieben, stellt sich bei einer Temperatur im Bereich von 70 - 90 °C und einem Druck von etwa 67 mbar eine Suspensionsdichte von 1,62 - 1,78 g/ml ein. Um ein gleichmäßiges Produkt zu erhalten und um eine gleichbleibende Produktausbeute erzielen zu können, ist es wichtig, während der Durchführung der Kristallisation darauf zu achten, dass die Suspensionsdichte eingehalten wird und die Temperatur in dem vorgegebenen Bereich liegt. Optimale Ergebnisse werden unter diesen Bedingungen erhalten, wenn die Suspensionsdichte während der Kristallisation bei 1,70 g/ml liegt und die Temperatur bei 80 °C gehalten wird. Zur kontinuierlichen Durchführung der Kristallisation wird laufend vorbereitete Lauge nachgefüllt, die bei 65 °C eine Suspensionsdichte von etwa 1, 28 - 1,31 g/ml aufweist und wie die ursprünglich eingesetzte Lösung mit H₂O₂-Lösung, Aktivkohle und durch Filtrieren vorbehandelt worden ist.

Wird die Kristallisation bei niedrigerer Temperatur durchgeführt, ist die Suspensionsdichte bei einem höheren Wert einzustellen. Wird beispielsweise die Kristallisation bei Temperaturen im Bereich von 50 bis 60 °C, vorzugsweise bei 55 °C durchgeführt, ist die Dichte der Suspensionslösung möglichst im Bereich von 1,72 - 1,75 g/ml einzustellen. Es ist jedoch in jedem Fall ratsam, eine Suspensionsdichte im Bereich von 1,62 - 1,78 g/ml einzuhalten, um eine zügige Abtrennung des Kristallisats gewährleisten zu können.

Entsprechend der kontinuierlichen Verfahrensdurchführung wird die Mutterlauge im Kreis geführt und laufend mit Natriumhydrogencarbonat und Natronlauge aufgefüllt und, wie oben beschrieben, vorbehandelt, bevor die Lösung wieder zur Kristallisation eingesetzt wird. Es ist aber auch möglich, zum Teil mit entsprechender frisch hergestellter Lösung aufzufüllen, so dass Ansätze unter Zusatz von Mutterlauge in einer Menge von 0 bis 100% bezogen auf die Gesamtlösung kristallisiert werden.

Das sich bildende Kristallisat wird durch geeignete Vorrichtungen mittels Filtrieren, Zentrifugieren oder Abschleudern aus der Lösung abgetrennt. Beispielsweise kann der gebildete Kristallbrei mittels Mischerschnecke auf eine Siebschleuder (4-gängige Kratzerschnecke mit 0,25mm Siebeinsatz) gepumpt werden, wo das Produkt mit wenig Wasser gewaschen wird und über einen Stromtrockner in einen Mischtrockner eingefüllt wird. Unter laufender Durchmischung wird das erhaltene Produkt für mindestens 10 Stunden getrocknet und das Monohydrat in das gewünschte wasserfreie Natriumcarbonat überführt. Der verwendete Mischtrockner wird zu diesem Zweck mit etwa 1000 kg feuchtem, kristallinem Natriumcarbonat befüllt und die Heizleistung so eingestellt, dass sich eine Temperatur im Bereich von 115 bis 125 °C einstellt. Die Trocknung ist beendet, wenn das erhaltene Produkt in Form von wasserfreiem Natriumcarbonat einen Trocknungsverlust von nicht mehr als 0,2 Gew.-%, vorzugsweise weniger als 0,15 Gew.-% aufweist. Bei den im Mischtrockner vorgelegten Produktmengen ist das nach etwa 10 Stunden der Fall.

Statt in einem Mischtrockner kann die nachfolgende Trocknung unter Bildung des wasserfreien Natriumcarbonats auch in einem Fließbett erfolgen. Solch ein Fließbett kann verschiedene, dem Fachmann bekannte Ausgestaltungen aufweisen. Zur Durchführung des in dieser Beschreibung wiedergegebenen Beispiels 2 wurde ein 3-Zonen-Fließbetttrockner eingesetzt.

In dem beschriebenen Beispiel 1 erfolgt die Trocknung in einem beheizbaren Mischtrockner unter Vakuum (Vakuumtrockner). Bei dem Trockner handelt es sich um einen im Handel erhältlichen Konus-Schnecken-Trockner. Aufgrund der Abmessungen des Trockners und seiner Ausgestaltung sind ein gleichmäßiger Wärmeaustausch und eine fortwährende Produktdurchmischung während der Trocknung gewährleistet. In dem Vakuumtrockner erfolgt die Trocknung bei etwa 20 bis 60 mbar, vorzugsweise bei 35 bis 45 mbar. Die Trocknungstemperatur im Bereich von 115 bis 125 °C wird während des Trocknungsvorgangs mit indirekter Dampfbeheizung bei etwa 4 bar reguliert. Durch die Vakuumbeaufschlagung erfolgt in einer verkürzten Trocknungszeit eine besonders schonende Trocknung. Es können auch andere anders ausgestaltete Mischtrockner zu diesem Zweck eingesetzt werden, die denselben Zweck erfüllen und vorzugsweise kontinuierlich betrieben werden können.

Anders als in Beispiel 1 beschrieben erfolgt dagegen in Beispiel 2 die Endtrocknung in einem Fließbett mit Heißluft, und zwar derart, dass sich eine Temperatur im Bereich von 175 bis 200 °C einstellt. Vorzugsweise erfolgt die Trocknung bei einer Temperatur im Bereich von 180 bis 195 °C. In der speziellen Ausführung des Beispiels 2 stellt sich eine Temperatur von 187 °C ein. Bei dieser Temperatur wird ein Produkt mit den geschilderten verbesserten Eigenschaften erhalten, wie sie in den folgenden Tabellen für das Produkt des Beispiels 2 wiedergegeben sind. Das zur Durchführung des Beispiels 2 eingesetzte Fließbett ist ein 3-Zonen-Fließbetttrockner mit einer Länge von 4550 mm, einer Breite von 450 mm, einem Wehr in der 1. Zone von 130 mm und einem auslaufseitigen Wehr mit einer Abmessung von 200 mm. Zur Trocknung des Kristallisats können auch Fließbetttrockner mit anderen Abmessungen und Ausgestaltungen eingesetzt werden, wesentlich ist jedoch sowohl bei Trocknung im Mischertrockner als auch im Fließbetttrockner, dass die Trocknung und Kalzinierung in einem Schritt bei relativ schonenden Temperaturen und unter fortwährender Durchmischung durchgeführt werden kann. Im Fließbetttrockner ist dieses durch die während der Trocknung erfolgende ständige Verwirbelung der kristallinen Produktpartikel im heißen Gasstrom verwirklicht.
Nach dem Abkühlen des Produkts in der Abkühlzone bei einer Temperatur von etwa 24 °C wird ein als Produkt wasserfreies Natriumcarbonat erhalten, das einen Trocknungsverlust von nicht mehr als 0,2 Gew.-% aufweist. Vorzugsweise weist das Produkt einen Trocknungsverlust von weniger als 0,15 Gew.-% auf bei einem Gehalt an Natriumcarbonat von nicht weniger als 99,5 Gew.-%. Speziell für ein Produkt, welches entsprechend der Beschreibung von Beispiel 2 hergestellt ist, liegt der acidimetrisch bestimmte Gehalt an Natriumcarbonat im Bereich von 99,6 - 99,7 Gew.-%.

Erfindungsgemäß hergestelltes wasserfreies Natriumcarbonat weist besonders vorteilhafte Eigenschaften für die Weiterverarbeitung zu pharmazeutischen Formulierungen auf. Wie die gegebenen Beispiele 1 und 2 zeigen, haben erfindungsgemäße Produkte Schüttdichten im Bereich von 1,050 bis 1,140 g/ml, und weisen eine Stampfdichte im Bereich von 1,18 bis 1,30 g/ml auf. Weiterhin liegen Schüttwinkel der erfindungsgemäß hergestellten Produkte im Bereich von 30,0° bis 31,5°, wodurch sie sich besonders gut verarbeiten lassen oder gegebenenfalls nach vorheriger Kornfraktionierung durch Siebung oder Vermahlung in galenischen Zubereitungen in Form von festen pharmazeutischen Formulierungen, wie Tabletten, Kapseln, Pulver, Granulaten oder Kapseln zur oralen Einnahme einsetzen lassen. Hierin lassen sie sich wiederum besonders gut mit sauren oder sauer wirkenden Additiven und Wirkstoffen verarbeiten.

### Bedingungen zur Herstellung:

Die Bedingungen zur Herstellung des erfindungsgemäßen Produktes ergeben sich aus der folgenden Herstellbeschreibung in den Beispielen 1 und 2. Die Produkteigenschaften ergeben sich aus den gemessenen physikalischen Charakterisierungsdaten, die in den Tabellen 1 bis 6 zusammengefasst sind.

### Produkteigenschaften und deren Bestimmung

Die Eigenschaften des hier beschriebenen hochreinen Natriumcarbonats in der Verwendung als Tablettierhilfsmittel und als Bestandteil in pharmazeutischen Formulierungen werden insbesondere durch die Größe der Partikel, deren Aufbau und Oberflächenbeschaffenheit, aber auch durch den Wassergehalt bestimmt. Um ein lagerfähiges Produkt zu haben, das möglichst kein CO₂ freisetzt, ist es wichtig ein möglichst wasserfreies Produkt herzustellen. Für die Herstellung von tablettierten Produkten aus dem beschriebenen Natriumcarbonat sind die Schüttdichte, die Stampfdichte und der Schüttwinkel von erheblicher Bedeutung. Um über die Zeit immer wieder gleichbleibende Produkte herstellen zu können und die Eigenschaften dieses Ausgangsprodukts vergleichen zu können, sollten immer wieder dieselben Methoden zur Bestimmung eingesetzt werden. Daher werden auch im vorliegenden Fall zur Beurteilung der Eigenschaften normierte Verfahren zur Bestimmung der verschiedenen Produkteigenschaften eingesetzt.
1. Die Bestimmung der Schüttdichte gemäß erfolgt DIN EN ISO 60: 1999 (Deutsche Fassung); Angabe in den Tabellen als "g/ml".
2. Die Bestimmung der Stampfdichte wird gemäß DIN EN ISO 787-11: 1995 (Deutsche Fassung) durchgeführt - Angabe in den Tabellen als "g/ml"
3. Die Bestimmung des Schüttwinkels erfolgt gemäß DIN ISO 4324: 1983 (Deutsche Fassung); Angabe in den Tabellen als "Grad"
4. Die Oberfläche wird gemäß BET bestimmt und Durchführung und Auswertung erfolgt wie in der Literaturstelle "BET Surface Area by Nitrogen Absorption" von S.Brunauer et al. (Journal of American Chemical Society, 60, 9, 1983) beschrieben, wobei die Messungen mit dem Gerät: ASAP 2420 der Firma Micromeritics Instrument Corporation (USA) erfolgt, und zwar unter Stickstoff. Die Bestimmung erfolgt mit einer Einwaage von 3,0000g und einer Ausheizung bei 50°C (10 h). Bei den angegebenen Werten handelt es sich um den arithmetischen Mittelwert aus drei Bestimmungen.
5. Die Bestimmung des Porenvolumens erfolgt durch Quecksilberintrusion mit einem Gerät der Firma CE INSTRUMENTS (PASCAL 400), in einem Druckbereich von 0-70 atm für Poren mit einem Durchmesser von 200000-2000Ä und in einem Druckbereich von 70-2000 atm für Poren mit einem Durchmesser von 2000-36Ä unter Verwendung von Proben mit einer Einwaage von ca. 100 mg.
6. Die Partikelgrößenbestimmung erfolgt entweder
   a) über Laserbeugung mit Trockendispergierung mit dem Gerät Mastersizer 2000 mit der Dispergiereinheit Scirocco 2000 der Firma Malvern Instruments Ltd. (UK). Die Bestimmungen werden bei 1, 2 und 3 bar Gegendruck durchgeführt; Auswertung nach Fraunhofer;
      Auswertemodell: General purpose; Brechungsindex 1.000;
      Obscuration: 7 - 20 %; Messzeit (snaps bzw. ms) 7500;
      Zufuhrrate: 100 %.
      Die Durchführung der Messung erfolgt gemäß ISO 13320-1 sowie entsprechend den Angaben des technischen Handbuchs des Geräteherstellers;
      Angaben in Vol%
      oder
   b) über Laserbeugung mit Naßdispergierung mit dem Gerät Mastersizer 2000 mit der Naßdispergiereinheit Hydro 2000S der Firma Malvern Instruments Ltd. (UK). Als Dispersionsmedium wird Ethanol, vergällt mit 1 % Ethylmethylketon (Art. Nr. 1.00974 Merck KGaA, Deutschland) verwendet; Brechungsindex 1.360; Obscuration: 10 - 20 %; Messzeit (snaps bzw.ms) 7500; Rührerdrehzahl 2000 UPM; keine Ultraschallleistung. Vor der Messung wird die Probe für 3 Minuten in der Meßzelle unter Rühren bei 2000 UPM vordispergiert. Die Durchführung der Messung erfolgt gemäß ISO 13320-1, sowie entsprechenden Angaben des technischen Handbuchs des Geräteherstellers; Angaben in Vol.-%;
      oder
   c) durch Trockensiebung über einen Siebturm: Retsch AS 200 control der Firma Retsch (Deutschland). Zur Durchführung wird eine Substanzmenge von etwa 200,00 mg eingewogen; die Siebzeit: 30 Minuten; Intensität: 1mm; Intervall: 5 Sekunden. Es werden Analysensiebe mit Metalldrahtgewebe gemäß DIN ISO 3310; Siebweiten (in µm): 710, 600, 500, 400, 355, 300, 250, 200, 150, 100, 75, 50, 32 verwendet. Die Angabe der Mengenverteilung pro Siebfraktion in den Tabellen ist als "Gew.% der Einwaage" angegeben.
7. Zur Gehaltsbestimmung wird 1 g Material in 50 ml Wasser gelöst und mit HCl (1mol/L) gegen Methylorange zügig titriert. Nach dem Farbumschlag wird die Lösung zwei Minuten zum Sieden erhitzt, abgekühlt und, falls notwendig, nochmals bis zum Farbumschlag titriert.
   1 ml Salzsäure (1mol/L) entspricht 0,052995 g Natriumcarbonat. Der Gehalt wird auf getrocknete Substanz berechnet.
   Die prinzipielle Vorgehensweise zur Bestimmung ist in der Fachliteratur beschrieben wie z.B. in G.Jander, K.F.Jahr, H.Knoll "Maßanalyse - Theorie und Praxis der klassischen und der elektrochemischen Titrierverfahren", Verlag Walter de Gruyter, 1973 ISBN 3 11 005934 7
8. Die Bestimmung des Trocknungsverlusts erfolgt, indem 2g Substanz genau eingewogen werden und mindestens für zwei Stunden bei 300°C getrocknet werden, d. h. bis zur Gewichtskonstanz. Die Angabe des Gewichtsverlusts erfolgt in Gew.-%.
9. REM-Aufnahmebedingungen: Gerät LEO 1530 (Carl Zeiss, Oberkochen, Deutschland)
10. Sputter Coater FCD 050 (Balzers Union, Liechtenstein) oder EMITECH K575 (EM Technologies, Ashford (Kent), England). Die Proben werden mit einem Leittab fixiert und mit Platin in Argonatmosphäre gesputtert.
11. TGA-Bedingungen: Instrument: Auto TGA 2950 HR V5.4A, Methode: 20K / min., Res. 3, Sens. 5 -> 500, 74ml N₂ /min., Pt-Tiegel; Angabe Gewichtsverlust in Gew.%; Durchführung der Messung gemäß Herstellervorgaben.
12. DVS-Bedingungen: Surface Measurement Systems Ltd. UK 1996 - 2000, Method: 0 - 98%, 10% Steps, 25°C, 0,0005% min., Halfcycle.sao; Durchführung der Messung gemäß Herstellervorgaben
13. Dichtebestimmungen: in der Regel durch Stimmgabelmessungen, z. B. mit Geräten der Firma Anton Paar GmbH/Österreich; Ausnahme: die Dichtebestimmung im Kristallisator erfolgt durch einen Coriolis Massendurchflussmesser, z. B. von der Firma Endress + Hauser Messtechnik GmbH + Co. KG/Deutschland. Alle Messungen erfolgen gemäß der Durchführungsbeschreibungen der Gerätehersteller.

Die vorliegende Beschreibung ermöglicht es dem Fachmann die Erfindung umfassend anzuwenden. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, die zitierten Veröffentlichungen und Patentliteratur heranzuziehen.

Zum besseren Verständnis und zur Verdeutlichung der Erfindung werden im Folgenden zwei Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew. bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten daher %-Angaben als Gew.- oder mol-%, mit Ausnahme von Verhältnissen, die in Volumenangaben wiedergegeben sind.

Die in den Beispielen und der Beschreibung sowie in den Ansprüchen gegebenen Temperaturen gelten immer in °C.

### Beispiele

### Beispiel 1

### Reaktion:

NaHCO₃ + NaOH → Na₂CO₃ x H₂O

**Chemikalien:**

| | |
|---|---|
| Natriumhydrogencarbonat | 23,8 kmol; 2000 kg wässrige Lösung |
| Natronlauge 45% | 25,9 kmol, 2300 kg wässrige Lösung |
| Aktivkohle | 0,5 kg |
| Wasserstoffperoxid (30%ig) | 10 l |
| VE-Wasser | 3500 l |

### Produkt-Ausbeute: Natriumcarbonat, wasserfrei ≈ 1250 kg (ca. 47 % d. Th.)

Für die Durchführung sind folgende Apparaturen notwendig:

| |
|---|
| 1 Rohstoffaufgabestation |
| 2 Ansatzbehälter 10000 l |
| 1 Scheiblerfilter |
| 2 Polizeifilter |
| 2 Reinstfiltratbehälter 10000 l |
| 1 Schlaufenkristaller 6300 l |
| 1 Kondensatbehälter |
| 2 Mutterlaugesammelbehälter 5000 l |
| 1 Siebzentrifuge |
| 1 Förderschnecke |
| 1 Spülwasserauffangbehälter 500 l |
| 1 Stromtrockner |
| 1 Zyklon |
| 1 Filterbunker |
| 1 Mischtrockner 2500 l |
| 2 Abfüllung Fördereinrichtung |
| 1 Abluftwäscher |
| 2 Abwasserbehälter 10000 l |

Vor Beginn der Produktherstellung werden die Anlagenteile in geeigneter Weise miteinander verbunden und verschaltet, wobei eine wirksame Abluftbehandlung zu berücksichtigen ist. Da Natriumcarbonat, Natriumhydrogencarbonat und Natronlauge schwach wassergefährdend (WGK 1) sind, werden die Abluftbehandlungsanlagen, Wäscher und Filter über eine Partikelmessung im Abluftstrom überwacht. Die maximal zulässige Stoffmenge im Abluftstrom beträgt 20,00 mg/m³.

### Durchführung:

Einmalige Herstellung (Voransatz) einer Mutterlauge: Im Ansatzkessel werden ca. 3500 I kaltes VE-Wasser (vollentsalztes Wasser) oder Kondensat vorgelegt. Der Rührer wird eingeschaltet. Anschließend werden nacheinander 1550 l Natronlauge (45%ig), und anschließend langsam 2000 kg Natriumhydrogencarbonat eintragen (Dauer: ca. 2 Std. wegen starker Wärmeentwicklung). Der Ansatz wird auf etwa 80 °C erhitzt für etwa 10 Minuten. Nun werden etwa 10 - 15 l Wasserstoffperoxid (30 %ig) zugeben. Anschließend wird wieder auf 65°C abgekühlt. Die Lösung hat am Ende eine Dichte von ca. 1,28 - 1,31 g/ml bei ca. 65°C. Diese Einstellung ist zu überprüfen! Die anschließende Standzeit des Ansatzes muss mindestens 8 Stunden betragen.

Folgeansätze: Die aus dem Voransatz erhaltene Mutterlauge wird vorgelegt. Unter Rühren werden 1050 I Natronlauge (45 %ig) zugegeben. Langsam werden innerhalb einer Stunde 1500 kg Natriumhydrogencarbonat eingetragen und mit VE-Wasser auf 8000 I aufgefüllt und 12,5 I Wasserstoffperoxid (30 %ig) zugegeben und für 10 Minuten zum Sieden erhitzt. Der Ansatz wird auf 65 °C abgekühlt. Bei dieser Temperatur soll die Lösung eine Dichte von 1,28 - 1,31 g/ml haben. Die Standzeit des Ansatzes muss ebenfalls mindestens 8 Stunden betragen.

Im Anschluss an die Ruhezeit wird die Lösung nach Zugabe von Aktivkohle mit der vorgesehenen Filteranlage filtriert (Feinfilter 1,0 µm Filter), so dass eine klare Lösung erhalten wird.

Zur Kristallisation wird der Kristaller gefüllt und der Rührer, Kreislaufpumpe und Vakuumpumpe eingeschaltet (optimale Einstellung beachten). Die Bodenheizung und der mittlere Heizkreis aktiviert und die. Dampfventile am Wärmetauscher geöffnet.

Die Natriumcarbonatlösung wird füllstandsgeregelt dem Schlaufenkristaller zugeführt und kontinuierlich kristallisiert. Während der Kristallisation ist eine Suspensionsdichte im Bereich von 1,62-1,78 g/ml und eine Kristallisationstemperatur im Bereich von 70-90 °C einzuhalten. Das Kondensat geht anstelle von VE-Wasser in dafür vorgesehene Behälter entsorgt. Während der Kristallisation ist auf eine optimale Sollwerteinstellung zu achten.
Rührerdrehzahl ≈ 60 U/min
Behälterdruck ≈ 67 mbar
Füllstand ≈ 45 %
Dichte 1, 70 g/ml (Temperatur: 80°C optimale Einstellung)

Zur kontinuierlichen Durchführung der Kristallisation ist laufend entsprechend vorbereitete Lauge nachzufüllen, die bei 65°C eine Dichte von etwa 1,28 - 1,31 g/ml aufweist und wie die ursprünglich verwendete Mutterlauge vorbehandelt worden ist.

### Prüfung der zur Kristallisation eingesetzten Lauge:

Um die Einstellungen zu überprüfen, werden in einem sauberen Becherglas 20 ml Bariumchloridlösung (10 %ig) vorgelegt. Zu dieser Bariumchloridlösung werden dann 2 ml der zur Kristallisation verwendeten Ansatzlösung gegeben. Nach der Zugabe von 5 Tropfen Phenolphthaleinlösung färbt sich die Lösung rot. Die Lösung wird langsam mit 0,1 N Salzsäure bis zur Entfärbung titriert. Der Verbrauch an Salzsäure muss zwischen 6,0 und 7,0 ml liegen. Gegebenenfalls wird der Ansatz wie folgt korrigiert:
Die Ansatzlösung wird mit Wasserkühlung auf eine Temperatur von 40 °C gebracht.
Bei einem Verbrauch > 7,0 ml wird Natriumhydrogencarbonat zugegeben.

Bei einem Verbrauch < 6,0 ml wird Natronlauge zugegeben.

Der gebildete Kristallisatbrei wird mittels Mischerschnecke auf eine Siebschleuder (4-gängige Kratzerschnecke und 0,25 mm-Siebeinsatz) gepumpt und das Produkt mit ≈ 55 l/h VE-Wasser gewaschen.
Das feuchte Produkt wird über einen Stromtrockner (Ablufttemperatur im Bereich von 60 - 70 °C) in den Mischtrockner so eingefüllt, dass der Mischer mit ca 1000 kg Natriumcarbonat befüllt ist.
Im Mischertrockner wird das Produkt für 10 Stunden mit 4 bar indirekter Dampfheizung getrocknet, wobei sich eine Endtemperatur von ca. 115 - 125 °C bei 40 mbar Vakuum einstellt. Anschließend wird auf 30 °C abgekühlt. Das Abkühlen dauert ca. 3 Stunden. Der Trocknungsverlust des Produktes darf nicht höher als 0,2 Gew.-% betragen.

Die Mutterlauge wird im Kreis geführt und mit ≈ 1500 kg Natriumhydrogencarbonat und ca. 1050 l Natronlauge (45%ig) aufgefüllt, und wie unter "Folgeansätze" beschrieben, weiterverfahren. Es können Ansätze mit Mutterlauge von 0-100 % gefahren werden.

### Beispiel 2

Die Herstellung des Kristallisats erfolgt wie unter Beispiel 1 beschrieben, jedoch wird nun bei ca. 55°C und einer Dichte von 1,72 - 1,75 g/ml kristallisiert und über eine Schneckenförderung in einen 3-Zonen-Fließbetttrockner (z. B. von Vibra Maschinenfabrik Schultheis GmbH & Co.KG/Deutschland oder der Firma Hosokawa Micron Group/Japan) eingebracht und zum Natriumcarbonat wasserfrei getrocknet. (Das Fließbett hat dabei eine Länge von 4550 mm und eine Breite von 450 mm, das Wehr der Zone 1 eine Höhe von 130 mm und das Auslaufwehr eine Höhe von 200 mm.) Die Trocknung im Fließbett erfolgt in den Zonen 1 und 2 bei 187 °C bei mit einer anschließenden Abkühlzone bei 24 °C. Die eingesetzte Luftmenge in den Zonen 1 und 2 liegt bei 890 - 910 m³/h bei einem Massendurchsatz an Produkt von 2000 bis 6000 l/d (Liter pro Tag). Die Temperatur der Zuluft und die Verweilzeit des Materials in der Anlage müssen so gewählt werden, dass das Endprodukt nach dem Verlassen des Fließbetttrockners einen maximalen Trocknungsverlust von 0,2 Gew.-% aufweist.

Durch die Trocknung des Monohydrates im Fließbetttrockner ist es möglich, ein wasserfreies Natriumcarbonat kontinuierlich herzustellen, welches einen Gehalt von 99,6 - 99,7 % an Natriumcarbonat aufweist.

### Vergleich der Eigenschaften des erfindungsgemäß hergestellten wasserfreien Natriumcarbonats aus den Beispielen 1 und 2 mit handelsüblichen Mustern:

Vergleich 1: Natriumcarbonat, chem. rein, wasserfrei, pulv., Ph. Eur., NF, FCC, E500, #1034, Ch.:RBA0290700, Chemische Fabrik Lehrte Dr.Andreas Kossel GmbH, D-Lehrte/Germany
Vergleich 2: Sodium carbonate IPH, Ch.:DOC2040821, Ph. Eur., Solvay Chemicals International, Dombasle plant, F-Dombasle/Frankreich
Beispiel 1: erfindungsgemäßes Produkt nach Trocknung in einem VakuumKontakt-Trockner (Konus-Schnecken-Trockner)
Beispiel 2: erfindungsgemäßes Produkt nach Trocknung in einem Wirbelschicht-Trockner

**Tabelle 1:**

| **Schüttdichte, Stampfdichte, Schüttwinkel:** | | | |
|---|---|---|---|
| | Schüttdichte (g/ml) | Stampfdichte (g/ml) | Schüttwinkel (Grad) |
| Vergl. 1 | 0,721 | 0,906 | 33,0 |
| Vergl. 2 | 0,656 | 0,838 | 36,7 |
| Beisp. 1 | 1,066 | 1,208 | 30,5 |
| Beisp. 2 | 1,122 | 1,276 | 31,0 |

**Tabelle 2:**

| **Gehalt, Trocknungsverlust, TGA (Thermogravimetrie):** | | | |
|---|---|---|---|
| | Gehalt (Gew.%) | Trocknungsverlust (Gew.%) | TGA (Gew.%) |
| Vergleich 1 | 98,3 | 1,4 | 1,037 |
| Vergleich 2 | 98,6 | 1,3 | 1,117 |
| Beispiel 1 | 99,8 | 0,12 | 0,065 |
| Beispiel 2 | 99,7 | 0,20 | 0,161 |

**Tabelle 3:**

| **Oberfläche und Porenvolumen, bestimmt nach dem BET Verfahren, totales kumulatives Porenvolumen und Porendurchmesser, bestimmt durch Quecksilberintrusion** | | | | |
|---|---|---|---|---|
| | BET-Oberfläche (m²/g) | BET-Porenvolumen (cm³/g) | Totales kumulatives Vol. (mm³/g) | Durchschnittlicher Porendurchmesser (Å) |
| Vergl. 1 | 2,34 | ∼0,0079 | 353 | 4138 |
| Vergl. 2 | 2,22 | ∼0,0067 | 330 | 4068 |
| Beispiel 1 | ∼0,09 | ∼0,0008 | 151 | 56 |
| Beispiel 2 | ∼0,12 | ∼0,0013 | 85 | 38 |

**Tabelle 4:**

| **DVS-Messung (Dynamic Vapor Sorption):** | | | | | | | | | | | |
|---|---|---|---|---|---|---|---|---|---|---|---|
| | **Relative Feuchte (%)** | | | | | | | | | | |
| | **0,0** | **10,0** | **20,0** | **30,0** | **40,0** | **50,0** | **60,0** | **70,0** | **80,0** | **90,0** | **98,0** |
| Vergl. 1 | 0,0 | 0,0 | 0,0 | 0,1 | 0,1 | 1,0 | 10,3 | 14,9 | 29,9 | 60,0 | 111,7 |
| Vergl. 2 | 0,0 | 0,0 | 0,1 | 0,1 | 0,1 | 0,7 | 10,8 | 16,1 | 31,2 | 68,4 | 112,1 |
| Beisp. 1 | 0,0 | 0,0 | 0,0 | 0,0 | 0,0 | 0,0 | 1,2 | 3,4 | 8,5 | 30,4 | 71,0 |
| Beisp. 2 | 0,0 | 0,0 | 0,0 | 0,0 | 0,0 | 0,0 | 1,3 | 2,7 | 10,0 | 46,7 | 79,0 |

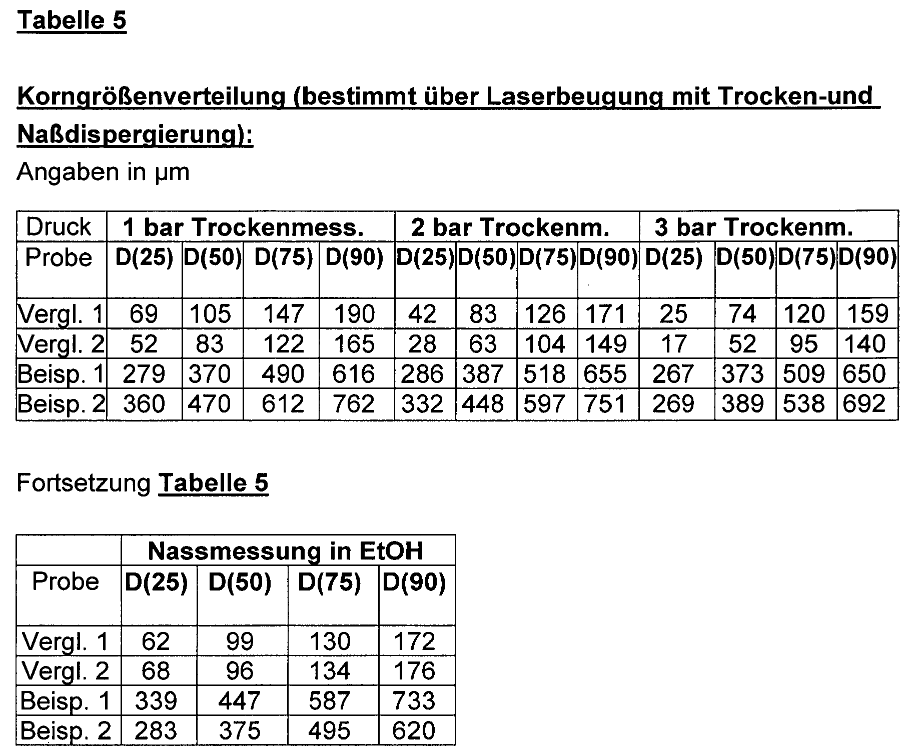

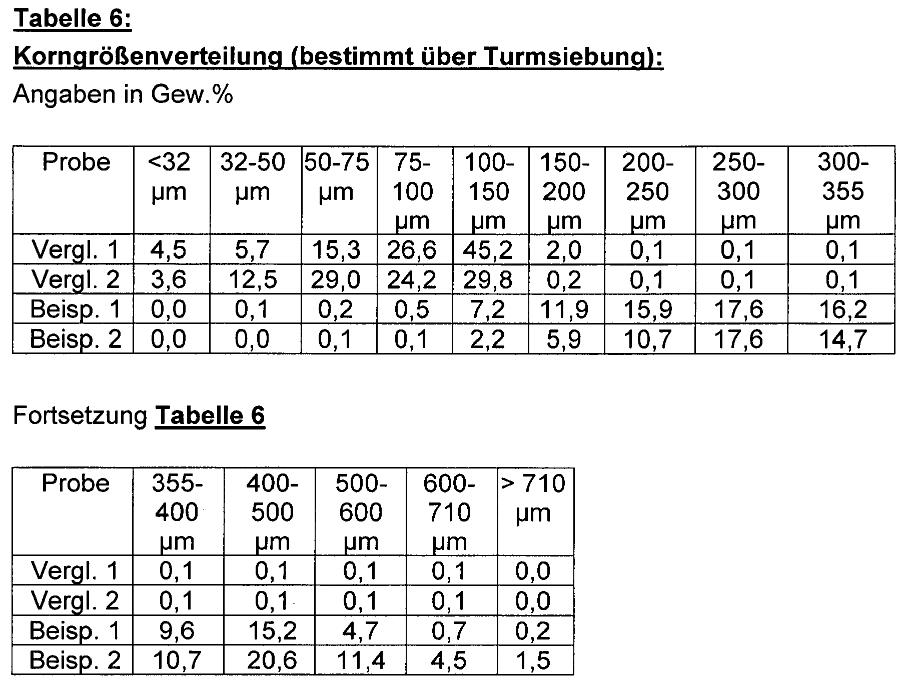

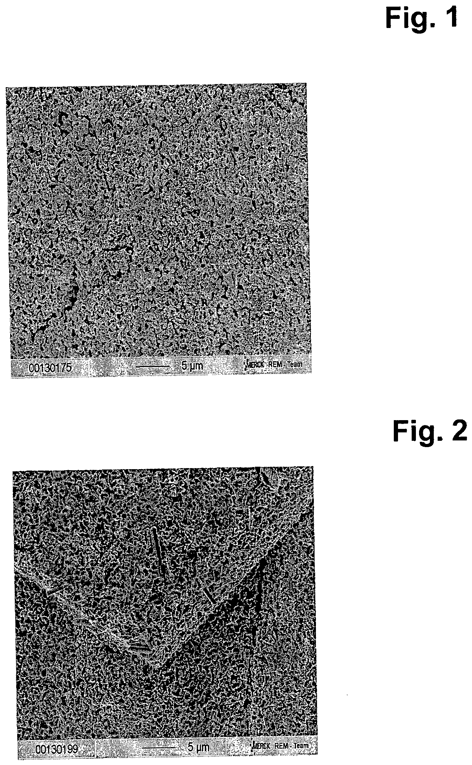

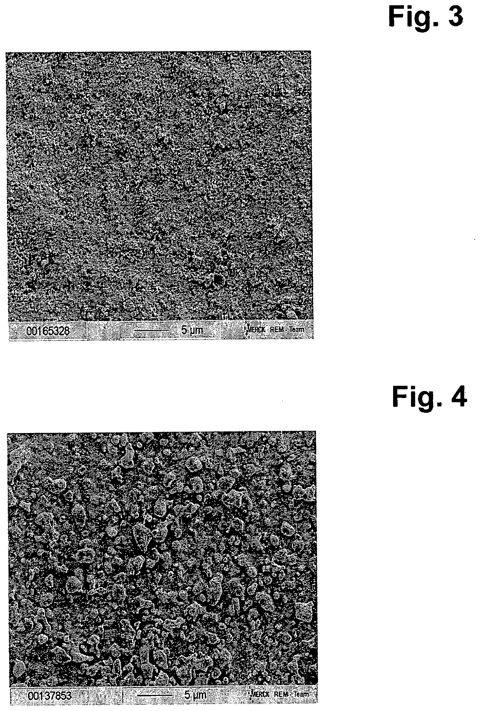

Auch in entsprechenden REM-Aufnahmen (siehe REM Aufnahmen (2500x) Fig. 1 -4) zeigt sich, dass erfindungsgemäß hergestelltes Natriumcarbonat (Beispiele 1 und 2) eine deutlich kompaktere (porenarme) Oberflächenstruktur aufweist als im Handel erhältliche Produkte (Vergleiche 1 und 2). Während die gemäß der Beispiele 1 und 2 (Fig. 3 und 4) hergestellten Materialien sehr kompakte Oberflächen aufweisen, sind in den Oberflächen der Vergleichsmaterialien 1 und 2 (Fig. 1 und 2) deutlich poröse Strukturen zu erkennen.

### Liste der Abbildungen:

**Fig.1****:**
   REM Aufnahme (2500x) von Natriumcarbonat, chemisch rein, wasserfrei, pulverförmig, Ph.Eur., NF, FCC, E500, #1034, Ch. RBA0290700, Chemische Fabrik Lehrte Dr. Andreas Kossel GmbH, D-Lehrte/ Germany
**Fig. 2****:**
   REM Aufnahme (2500x) von Sodium carbonate IPH, Ch.: DOC2040821, Ph. Eur., Solvay Chemicals International, Dombasle/ Frankreich
**Fig. 3****:**
   REM Aufnahme (2500x) einer Probe eines erfindungsgemäß nach Beispiel 1 hergestellten, wasserfreien Natriumcarbonats
**Fig. 4****:**
   REM Aufnahme (2500x) einer Probe eines erfindungsgemäß nach Beispiel 2 hergestellten, wasserfreien Natriumcarbonats

## Patentansprüche

1. Kristallines Natriumcarbonat, **dadurch gekennzeichnet, dass** es ein wasserfreies, porenarmes, hochreines Produkt ist, welches einen Gehalt an Natriumcarbonat von nicht weniger als 99,5% aufweist (acidimetrisch bestimmt) und
a) aus Teilchen mit einer glatten, porenarmen Oberflächenstruktur besteht,
b) eine BET-Oberfläche von kleiner 1m²/g, vorzugsweise von kleiner 0.5 m²/g, insbesondere bevorzugt von kleiner 0,2 m²/g besitzt
und
c) nach seiner Herstellung einen Trocknungsverlust von nicht mehr als 0,2 Gew.-%, vorzugsweise von weniger als 0,15 Gew.-% aufweist.

2. Wasserfreies Natriumcarbonat gemäß Anspruch 1, **dadurch gekennzeichnet, dass** es ein Wasserdampfaufnahmevermögen (DVS) von <5 Gew.-% bei 60% relative Feuchte; < 10 Gew.-% bei 70% relative Feuchte; <15 Gew.-% bei 80% relative Feuchte und < 50% bei 90% relative Feuchte aufweist.

3. Wasserfreies Natriumcarbonat gemäß der Ansprüche 1, 2, oder 3, **dadurch gekennzeichnet, dass** es eine Schüttdichte im Bereich von 1,050 bis 1,140 g/ml, eine Stampfdichte im Bereich von 1,18 bis 1,30 g/ml und einen Schüttwinkel im Bereich von 30,0° bis 31,5°besitzt.

4. Verfahren zur Herstellung eines wasserfreien Natriumcarbonats gemäß der Ansprüche 1 - 3, **dadurch gekennzeichnet, dass**
a) eine wässrige Lösung hergestellt wird, worin Natriumhydrogencarbonat und Natronlauge in einer solchen Menge gelöst sind, dass die Lösung bei 65 °C eine Dichte im Bereich von 1,2 bis 1,4 g/ml, bevorzugt im Bereich von 1,25 bis 1,34 g/ml, besonders bevorzugt Bereich von 1,28 bis 1,31 g/ml aufweist und Natriumhydrogencarbonat (NaHCO₃) und Natronlauge (NaOH) darin in einem molaren Verhältnis von 1 : 1 bis 1 : 1,3 enthalten sind, anschließend
b) die Lösung nacheinander mit einer katalytischen Menge Wasserstoffperoxid und mit Aktivkohle versetzt wird und nach ausreichender Durchmischung filtriert wird,
und
c) die erhaltene klare Lösung einer Kristallisationsapparatur zugeführt wird, worin durch Vakuumverdampfung bei erhöhter Temperatur Flüssigkeit entfernt wird und eine Suspensionsdichte im Bereich von 1,54 - 1,86 g/ml, bevorzugt 1, 58 - 1,82 g/ml, besonders bevorzugt im Bereich von 1,62 - 1,78 g/ml eingestellt wird, bei der die Kristallisation erfolgt,
e) die gebildeten Kristalle durch Filtrieren, Zentrifugieren oder Abschleudern abgetrennt werden
und
f) in einen Trockner gefüllt werden und unter fortwährender Durchmischung bei erhöhter Temperatur getrocknet werden bis das erhaltene Produkt einen Trocknungsverlust von nicht mehr als 0,2 Gew.-% aufweist.

5. Verfahren gemäß Anspruch 4, **dadurch gekennzeichnet, dass** es in einem Schlaufenkristaller durchgeführt wird.

6. Verfahren gemäß Anspruch 4 oder 5, **dadurch gekennzeichnet, dass** es batchweise oder kontinuierlich durchgeführt wird.

7. Verfahren gemäß Anspruch 4, 5 oder 6, **dadurch gekennzeichnet, dass** die Temperatur während der Kristallisation im Bereich von 50 bis 95 °C, vorzugsweise im Bereich von 55 bis 90 °C, eingestellt wird, während die Dichte der Suspension im Bereich von 1,54 - 1,86, vorzugsweise im Bereich von 1,62 bis 1,78 g/ml, liegt.

8. Verfahren gemäß Anspruch 4, 5, 6 oder 8, **dadurch gekennzeichnet, dass** die in Schritt a) hergestellte Lösung mit Wasserstoffperoxid (30%ig) in einem Verhältnis 700 : 1 bis 900 :1, vorzugsweise 800 :1 bis 760:1, insbesondere bevorzugt 780 :1, bezogen auf das Gewicht der Gesamtlösung und mit Aktivkohle in einem Verhältnis 20000 :1 bis 10000 : 1, vorzugsweise 16500 :1 bis 14000 : 1, insbesondere bevorzugt 15600 :1 bezogen auf das Gewicht der Gesamtlösung, versetzt wird.

9. Verfahren gemäß einem oder mehreren der Ansprüche 4 bis 8, **dadurch gekennzeichnet, dass** die Kristallisation bei einer Temperatur im Bereich von 70 bis 90 °C und einer Suspensionsdichte im Bereich von 1,62 - 1,78 g/ml durchgeführt wird.

10. Verfahren gemäß einem oder mehreren der Ansprüche 4 bis 9, **dadurch gekennzeichnet, dass** die Kristallisation bei einer Temperatur im Bereich von 50 bis 60 °C, vorzugsweise bei 55 °C und einer Suspensionsdichte im Bereich von 1,72 bis 1,75 g/ml durchgeführt wird.

11. Verfahren gemäß einem oder mehreren der Ansprüche 4 bis 10, **dadurch gekennzeichnet, dass** das Kristallisat mittels Mischerschnecke auf eine Siebschleuder geführt, mit Wasser gewaschen und über einen Stromtrockner in einen Mischertrockner gefüllt wird und unter fortwährender Durchmischung bei einer Temperatur 115 bis 125 °C getrocknet werden bis das erhaltene Produkt einen Trocknungsverlust von weniger als 0,2 Gew.-% aufweist.
oder
nach dem Abschleudern über eine Schneckenförderung in einen Fließbetttrockner geführt und bei einer Temperatur im Bereich von 175 bis 200 °C, vorzugsweise im Bereich von 180 bis 195 °C, insbesondere bevorzugt bei 187°C, mit einer ausreichenden Verweilzeit getrocknet wird, so dass das erhaltene Produkt einen Trocknungsverlust von nicht mehr als 0,2 Gew.-% aufweist.

12. Verwendung von wasserfreiem Natriumcarbonat gemäß der Ansprüche 1 bis 3 in galenischen Zubereitungen, gegebenenfalls nach vorheriger Kornklassifizierung durch Siebung oder Vermahlung, in Form von festen pharmazeutischen Formulierungen, wie Tabletten, Kapseln, Pulver, Granulaten oder Kapseln zur oralen Einnahme.

13. Verwendung von wasserfreiem Natriumcarbonat gemäß der Ansprüche 1 bis 3 in Brauseformulierungen enthaltend Vitamine und/oder Mineralstoffe und/oder Spurenelemente und/oder hydrolyseempfindliche Wirkstoffe.

14. Verwendung von wasserfreiem Natriumcarbonat gemäß der Ansprüche 1 bis 3 in Tabletten, Granulaten oder Brauseformulierungen enthaltend sauer wirkende Wirkstoffe und/oder hydrolyseempfindliche Wirkstoffe.

## Claims

1. Crystalline sodium carbonate, **characterised in that** it is an anhydrous, low-pore, highly pure product which has a content of sodium carbonate of not less than 99.5% (determined acidimetrically) and
a) consists of particles having a smooth, low-pore surface structure,
b) has a BET surface area of less than 1 m²/g, preferably less than 0.5 m²/g, particularly preferably less than 0.2 m²/g
and
c) has a drying loss after preparation of not more than 0.2% by weight, preferably less than 0.15% by weight.

2. Anhydrous sodium carbonate according to Claim 1, **characterised in that** it has a water vapour absorption capacity (WVAC) of < 5% by weight at 60% relative humidity; < 10% by weight at 70% relative humidity; < 15% by weight at 80% relative humidity and < 50% at 90% relative humidity.

3. Anhydrous sodium carbonate according to Claim 1, 2 or 3, **characterised in that** it has a bulk density in the range from 1.050 to 1.140 g/ml, a tapped density in the range from 1.18 to 1.30 g/ml and an angle of repose in the range from 30.0° to 31.5°.

4. Process for the preparation of an anhydrous sodium carbonate according to Claims 1 - 3, **characterised in that**
a) an aqueous solution is prepared in which sodium hydrogencarbonate and sodium hydroxide solution are dissolved in such an amount that the solution has a density in the range from 1.2 to 1.4 g/ml, preferably in the range from 1.25 to 1.34 g/ml, particularly preferably in the range from 1.28 to 1.31 g/ml, at 65°C and sodium hydrogencarbonate (NaHCO₃) and sodium hydroxide solution (NaOH) are present therein in a molar ratio of 1 : 1 to 1 : 1.3, subsequently
b) a catalytic amount of hydrogen peroxide and activated carbon are added successively to the solution, which is then filtered after adequate mixing,
and
c) the clear solution obtained is fed to a crystallisation apparatus, in which liquid is removed by vacuum evaporation at elevated temperature and a suspension density in the range from 1.54 - 1.86 g/ml, preferably 1.58 - 1.82 g/ml, particularly preferably in the range from 1.62 - 1.78 g/ml is set at which the crystallisation is carried out,
e) the crystals formed are separated off by filtration, centrifugation or spinning-off
and
f) are introduced into a dryer and are dried at elevated temperature with continued mixing until the product obtained has a drying loss of not more than 0.2% by weight.

5. Process according to Claim 4, **characterised in that** it is carried out in a loop crystalliser.

6. Process according to Claim 4 or 5, **characterised in that** it is carried out batchwise or continuously.

7. Process according to Claim 4, 5 or 6, **characterised in that** the temperature during the crystallisation is set in the range from 50 to 95°C, preferably in the range from 55 to 90°C, while the density of the suspension is in the range from 1.54 - 1.86, preferably in the range from 1.62 to 1.78 g/ml.

8. Process according to Claim 4, 5, 6 or 8, **characterised in that** the solution prepared in step a) is mixed with hydrogen peroxide (30%) in a ratio of 700 : 1 to 900 :1, preferably 800 :1 to 760:1, particularly preferably 780 :1, based on the weight of the entire solution, and with activated carbon in a ratio of 20000 :1 to 10000 : 1, preferably 16500 :1 to 14000 : 1, particularly preferably 15600 :1, based on the weight of the entire solution.

9. Process according to one or more of Claims 4 to 8, **characterised in that** the crystallisation is carried out at a temperature in the range from 70 to 90°C and a suspension density in the range from 1.62 - 1.78 g/ml.

10. Process according to one or more of Claims 4 to 9, **characterised in that** the crystallisation is carried out at a temperature in the range from 50 to 60°C, preferably at 55°C, and a suspension density in the range from 1.72 to 1.75 g/ml.

11. Process according to one or more of Claims 4 to 10, **characterised in that** the crystals are fed to a screen centrifuge by means of a mixer screw, washed with water and introduced into a mixer dryer via a flow dryer and dried at a temperature of 115 to 125°C with continued mixing until the product obtained has a drying loss of less than 0.2% by weight.
or
after spinning-off are fed to a fluidised-bed dryer via a screw conveyor and dried at a temperature in the range from 175 to 200°C, preferably in the range from 180 to 195°C, particularly preferably at 187°C, with an adequate residence time, so that the product obtained has a drying loss of not more than 0.2% by weight.

12. Use of anhydrous sodium carbonate according to Claims 1 to 3 in pharmaceutical preparations, optionally after prior grain classification by sieving or grinding, in the form of solid pharmaceutical formulations, such as tablets, capsules, powders, granules or capsules for oral use.

13. Use of anhydrous sodium carbonate according to Claims 1 to 5 in effervescent formulations comprising vitamins and/or mineral substances and/or trace elements and/or hydrolysis-sensitive active compounds.

14. Use of anhydrous sodium carbonate according to Claims 1 to 3 in tablets, granules or effervescent formulations comprising acidic active compounds and/or hydrolysis-sensitive active compounds

## Revendications

1. Carbonate de sodium cristallin, **caractérisé en ce qu'**il s'agit d'un produit anhydre, peu poreux et hautement pur, qui possède une teneur en carbonate de sodium non inférieure à 99,5% (déterminé par voie acidimétrique) et
a) est constitué de particules ayant une structure de surface lisse et peu poreuse,
b) possède une surface BET inférieure à 1 m²/g, préférablement inférieure à 0,5 m²/g, particulièrement préférablement inférieure à 0,2 m²/g
et
c) possède une perte au séchage après préparation d'au plus 0,2% en poids, préférablement inférieure à 0,15% en poids.

2. Carbonate de sodium anhydre selon la revendication 1, **caractérisé en ce qu'**il possède une capacité d'absorption de vapeur d'eau (WVAC) < 5% en poids à 60% d'humidité relative ; < 10% en poids à 70% d'humidité relative ; < 15% en poids à 80% d'humidité relative et < 50% à 90% d'humidité relative.

3. Carbonate de sodium anhydre selon la revendication 1, 2 ou 3, **caractérisé en ce qu'**il possède une densité apparente dans la plage allant de 1,050 à 1,140 g/ml, une densité tassée dans la plage allant de 1,18 à 1,30 g/ml et un angle de repos dans la plage allant de 30,0° à 31,5°.

4. Procédé de préparation d'un carbonate de sodium anhydre selon les revendications 1 - 3, **caractérisé en ce que**
a) on prépare une solution aqueuse dans laquelle de l'hydrogéno-carbonate de sodium et une solution d'hydroxyde de sodium sont solubilisés selon une quantité telle que la solution possède une densité dans la plage allant de 1,2 à 1,4 g/ml, préférablement dans la plage allant de 1,25 à 1,34 g/ml, particulièrement préférablement dans la plage allant de 1,28 à 1,31 g/ml, à 65°C et l'hydrogénocarbonate de sodium (NaHCO₃) et la solution d'hydroxyde de sodium (NaOH) y sont présents selon un rapport molaire allant de 1:1 à 1:1,3, puis
b) une quantité catalytique de peroxyde d'hydrogène ainsi que de charbon actif sont ajoutées successivement à la solution, qui est ensuite filtrée après un mélange adéquat,
et
c) la solution limpide obtenue est alimentée dans un appareil de cristallisation, dans lequel le liquide est éliminé par évaporation sous vide à une température élevée et une densité de suspension dans la plage de 1,54 - 1,86 g/ml, préférablement de 1,58 - 1,82 g/ml, particulièrement préférablement dans la plage de 1,62 - 1,78 g/ml est réglée, à laquelle la cristallisation est effectuée,
e) les cristaux formés sont séparés par filtration, centrifugation ou essorage
et
f) sont introduits dans un séchoir et sont séchés à une température élevée sous mélange continu jusqu'à ce que le produit obtenu ait une perte au séchage d'au plus 0,2% en poids.

5. Procédé selon la revendication 4, **caractérisé en ce qu'**il est effectué dans un cristalliseur en boucle.

6. Procédé selon la revendication 4 ou 5, **caractérisé en ce qu'**il est effectué en mode discontinu ou continu.

7. Procédé selon la revendication 4, 5 ou 6, **caractérisé en ce que** la température pendant la cristallisation est réglée dans la plage allant de 50 à 95°C, préférablement dans la plage allant de 55 à 90°C, tandis que la densité de la suspension se trouve dans la plage de 1,54 - 1,86, préférablement dans la plage allant de 1,62 à 1,78 g/ml.

8. Procédé selon la revendication 4, 5, 6 ou 8, **caractérisé en ce que** la solution préparée dans l'étape a) est mélangée avec du peroxyde d'hydrogène (30%) selon un rapport allant de 700:1 à 900:1, préférablement de 800:1 à 760:1, particulièrement préférablement de 780:1, sur la base du poids de l'ensemble de la solution, et avec du charbon actif selon un rapport allant de 20 000:1 à 10 000:1, préférablement de 16 500:1 à 14 000:1, particulièrement préférablement de 15 600:1, sur la base du poids de l'ensemble de la solution.

9. Procédé selon l'une ou plusieurs parmi les revendications 4 à 8, **caractérisé en ce que** la cristallisation est effectuée à une température dans la plage allant de 70 à 90°C et une densité de suspension dans la plage de 1,62 - 1,78 g/ml.

10. Procédé selon l'une ou plusieurs parmi les revendications 4 à 9, **caractérisé en ce que** la cristallisation est effectuée à une température dans la plage allant de 50 à 60°C, préférablement à 55°C, et une densité de suspension dans la plage allant de 1,72 à 1,75 g/ml.

11. Procédé selon l'une ou plusieurs parmi les revendications 4 à 10, **caractérisé en ce que** les cristaux sont alimentés dans une essoreuse à l'aide d'une vis à mélangeur, lavés par de l'eau et introduits dans un mélangeur-sécheur via un séchoir à flux et séchés à une température allant de 115 à 125°C sous mélange continu jusqu'à ce que le produit obtenu ait une perte au séchage inférieure à 0,2% en poids.
ou
après essorage, sont alimentés vers un séchoir à lit fluidisé via un transporteur à vis et séchés à une température dans la plage allant de 175 à 200°C, préférablement dans la plage allant de 180 à 195°C, particulièrement préférablement à 187°C, avec un temps de séjour adéquat, de façon à ce que le produit obtenu ait une perte au séchage d'au plus 0,2% en poids.

12. Utilisation de carbonate de sodium anhydre selon les revendications 1 à 3 dans des préparations pharmaceutiques, éventuellement après une classification préalable des grains par tamisage ou broyage, sous la forme de formulations pharmaceutiques solides, telles que des comprimés, des capsules, des poudres, des granulés ou des capsules pour une utilisation orale.

13. Utilisation de carbonate de sodium anhydre selon les revendications 1 à 5 dans des formulations effervescentes comprenant des vitamines et/ou des substances minérales et/ou des oligo-éléments et/ou des composés actifs sensibles à l'hydrolyse.

14. Utilisation de carbonate de sodium anhydre selon les revendications 1 à 3 dans des comprimés, des granulés ou des formulations effervescentes comprenant des composés actifs acides et/ou des composés actifs sensibles à l'hydrolyse.
